Europäisches Patentamt

European Patent Office

Office européen des brevets

(11) Publication number: **0 208 539**
**A2**

(12) **EUROPEAN PATENT APPLICATION**

(21) Application number: 86305272.6

(22) Date of filing: 08.07.86

(51) Int. Cl.⁴: **C07K 15/00** , **C07K 15/12** , **C12P 21/00** , **C07K 7/00**

(30) Priority: 11.07.85 US 753848
23.12.85 US 812162
05.05.86 US 859595

(43) Date of publication of application:
14.01.87 Bulletin 87/03

(84) Designated Contracting States:
BE CH DE FR GB IT LI NL SE

(71) Applicant: REPLIGEN CORPORATION
101 Binney Street
Cambridge Massachusetts 02142(US)

(72) Inventor: Piglet, Vincent P.
666 Main Street Apt. L-1
Winchester Massachusetts 01898(US)
Inventor: Schuster, Barbara J.
3520 Plaza Drive
State College Pennsylvania 16801(US)

(74) Representative: Perry, Robert Edward et al
GILL JENNINGS & EVERY 53-64 Chancery
Lane
London WC2A 1HN(GB)

(54) **Folding disulfide-cross-linkable proteins.**

(57) A process for producing a folded, disulfide-cross-linked protein, in vitro, comprises either reacting the corresponding unfolded, reduced protein with an oxidised dithiol peptide selected from oxidised thioredoxin and derivatives and analogues thereof, or reacting a scrambled protein containing incorrect disulfide cross-links with a reduced dithiol peptide selected from reduced thioredoxin and derivatives and analogues thereof.

# FOLDING DISULFIDE-CROSS-LINKABLE PROTEINS

This invention relates to a process for folding proteins, to give folded proteins containing disulfide cross-links, in vitro.

A critical feature of a biologically-active protein is its conformation. When proteins are synthesised in vitro, they do not have the structure characteristic of the native, folded protein. Finding the correct "native" conformation is to a degree a matter of trial and error; however, the relatively rapid folding of proteins in vivo implies that there is a pathway of folding involving only relatively few intermediate species. Overall, the folding process is driven by the greater thermodynamic stability of the native form.

Under normal conditions in vivo, folding occurs very rapidly. In bacteria, refolding typically occurs in a few minutes.

The recent developments of recombinant technologies permit considerable amplification of the amount of specific proteins synthesised, but the folding of proteins can be slow. This often results in inclusion bodies containing unfolded, or partially folded, protein.

For proteins containing disulfide crosslinks - (disulfide proteins) this problem is particularly severe since bacteria, such as Escherichia coli, which are commonly used as a recombinant host, are not adapted to the processes of sulfhydryl oxidation to the disulfide form.

A number of studies of refolding of disulfide proteins have clarified the folding process as one of the successive formation (i.e., disulfide interchange) of specific disulfides. Kinetically, the efficiency of the overall process is limited by this interchange step. Several studies using low molecular weight thiols to catalyze this process (i.e., cysteine, glutathione) have defined optimal concentrations of oxidized and reduced thiol compounds. Rates observed are greatly improved relative to air oxidation, but such conditions still leave much to be desired for industrial scale processes. Such processes require obtaining adequate yields at reasonable times and protein concentrations. Thus, the goal in a protein folding process is to effectively fold the protein by not only increasing the rate of the reaction, but also by using greater protein concentrations without sacrificing yields.

Perhaps the most commercially important protein containing disulfide bonds is insulin. U.S. Patent 4,421,685 discloses a process for producing insulin wherein the S-sulfonated form of the A-chain and the S-sulfonated form of the B-chain are reacted with a thiol reducing agent under specific conditions to form insulin. Thiol reducing agents disclosed therein are dithiothreitol (DTT) and dithioerythritol (DTE).

The use of S-sulfonated proteins in the refolding of recombinant proteins is a common industrial process. (See U.S. Patent 4,421,685.) The use of sulfonated proteins allows for greater solubility and protects sulfhydryl groups during protein purification. In the presence of a limited amount of reductant, refolding is efficient with the protein thiosulfonate acting as a mixed disulfide intermediate. Most of the described processes use DTT, glutathione, or $\beta$-mercaptoethanol as the reductant.

Brief Summary of the Invention

The subject invention concerns an improved process for folding proteins containing disulfide crosslinks. The process has particular utility in recombinant DNA processes wherein proteins, or portions thereof, are produced by constructed microbes. The invention process comprises reacting an unfolded and reduced protein with an effective protein folding amount of oxidized thioredoxin, or an oxidized thioredoxin-derived, or thioredoxin-like, dithiol peptide, alone, or in combination with oxygen, or a thioredoxin reductase regenerating system, to produce folded protein. The invention process also comprises reacting a scrambled protein where incorrect disulfide bonds have been formed - (i.e., the protein is folded but in the wrong conformation, with only a fraction being active) with an effective concentration of oxidized and reduced thioredoxin, or oxidized thioredoxin and a chemical reducing agent such as dithiothreitol.

Detailed Disclosure of the Invention

Unfolded protein containing disulfide crosslinks can be efficiently folded by reacting the unfolded and reduced protein with an effective protein folding amount of oxidized thioredoxin, or an oxidized thioredoxin-derived, or thioredoxin-like, dithiol peptide, alone, or in combination with oxygen or a thioredoxin reductase regenerating system. Scrambled proteins can be correctly folded by reacting them with an effective protein folding amount of reduced thioredoxin or a reduced thioredoxin-derived, or thioredoxin-like, dithiol peptide, alone, or in combination with oxidized thioredoxin or an oxidized thioredoxin-derived, or thioredoxin-like, dithiol peptide.

The unfolded or scrambled protein can be in a crude cell extract, or partially purified from a crude cell extract, for example, from a recombinant organism.

The concentration of thioredoxin or one of the thioredoxin-derived, or thioredoxin-like, dithiol peptides which can be used in the invention process ranges from about 5 to about 500 $\mu$M. The optimal concentration for oxidized intact bacterial thioredoxin appears to be at least 500 $\mu$M and 10 $\mu$M for reduced thioredoxin. It should be recognized that the precise level of thioredoxin or thioredoxin-derived, or thioredoxin-like, dithiol peptide can be readily ascertained for a particular protein by a person skilled in the protein art having possession of the subject invention.

Thioredoxin at a concentration of 5-500 $\mu$M catalyzes the refolding of denatured and reduced bovine pancreatic RNase (22-160 $\mu$M) to give quantitative recovery of enzymatic activity. The rate of refolding was approximately fifteen-fold greater than air oxidation. With thioredoxin, 50% activity was recovered after 30 hr with 100 $\mu$M and 8 hr with 500 $\mu$M compared to 120 hr required using air oxidation. Thioredoxin was 1000 times more effective than oxidized dithiothreitol. Thioredoxin at $\mu$M concentrations resulted in the same kinetics of refolding as mM concentrations of oxidized DTT. When DTT (20 mM) and various concentrations of thioredoxin were added to the sample, there was an additional increase in the appearance of activity. This increase was constant for all thioredoxin concentrations, suggesting that the effects are synergistic.

The time required for 50% reactivation of RNase ($T_{1/2}$) in the presence of 100 $\mu$M thioredoxin was decreased three fold with the addition of oxygen. The $T_{1/2}$ for 100 $\mu$M thioredoxin and air was 23 hr. With the addition of oxygen the $T_{1/2}$ was lowered to 8 hr. This is comparable to the $T_{1/2}$ of 500 $\mu$M thioredoxin in the presence of air. The effective concentration of thioredoxin can be reduced by a factor of five with the addition of oxygen.

The oxygen effect does not occur in the absence of thioredoxin. There is an initial increase in the rate of refolding without thioredoxin, but the recovered activity levels off at 12% after 5 hr.

In the presence of a regenerating system of thioredoxin reductase and a nicotinamide adenine dinucleotide derivative, for example, NADP+ -(oxidized form), there was an increase in the rate of refolding of RNase with 100 $\mu$M thioredoxin. The $T_{1/2}$ was decreased from 23 hr to 5-6 hr with thioredoxin reductase and either 0.1 or 1.0 mM NADP+.

Thioredoxin efficiently catalyzes the rearrangement of disulfide bonds in scrambled, inactive RNase at pH 7.4 to yield a quantitative recovery of active enzyme. Reduced thioredoxin at a concentration of 10 $\mu$M had a $T_{1/2}$ of 8 hr. Optimal conditions for reactivating scrambled RNase consisted of a mixture of oxidized and reduced thioredoxin at a ratio of 10:1, respectively. The $T_{1/2}$ values for 100 $\mu$M, 150 $\mu$M and 200 $\mu$M thioredoxin at a ratio of 10:1 oxidized to reduced were 5, 2 and 1.5 hr, respectively. Neither air nor DTT alone were effective in reactivating scrambled RNase, with a recovery of less than 30% activity.

Thioredoxins are low molecular weight dithiol proteins that have the ability to reduce disulfides in typical organic compounds such as Elman's reagent or disulfides as they exist naturally in a variety of proteins (Holmgren, A. [1981] Trends in Biochemical Science, 6, 26-39).

Thioredoxin and thioredoxin-derived, or thioredoxin-like, dithiol peptides within the scope of the subject invention are exemplified by the following compounds:

(1) thioredoxin isolated from Escherichia coli (Laurent, T.C., Moore, E.C., and Reichard, P. - [1964] J. Biol. Chem., 239, 3436-3445);

(2) thioredoxins isolated from other sources, e.g., thioredoxin isolated from yeast (Porque, G.P., Baldesten, A., and Reichard, P. [1970] J. Biol. Chem., 245, 2362-2379); Cyanobacterium - (Gleason, F.K. and Holmgren, A. [1983] in "Thioredoxins, Structure and Function" [P. Gadal, ed.] Editions du Centre National de la Recherche Scientifique); rat (Guerara, J., Moore, E.C., and Ward, D.NM. [1983] ibid; T4 bacteriophage - (Soderberg, B-O, Sjoberg, B-M, Sonnerstam, U., and Branden, C-I [1978] Proc. Natl. Acad. Sci. USA, 75, 5827-5830);

(3) thioredoxin-derived dithiol peptides representing peptides produced by cleavage of intact thioredoxins, as described in Example 2, infra. One such example of this class of thioredoxin-derived peptides is the fragment containing residues 1 through 37 (i.e., $T_{1-37}$) produced by cyanogen bromide cleavage of thioredoxin from E. coli . The important feature of these thioredoxin-derived, and thioredoxin-like, dithiol peptides is that they contain the redox-active peptide sequence, Cys-X-Y-Cys-Lys, wherein X and Y can be any of the 20 amino acids. For example, the redox-active peptide sequence from E. coli thioredoxin is Cys-Gly-Pro-Cys-Lys (Cys = cysteine, Gly = glycine, Pro = proline, Lys = lysine); and

(4) thioredoxin-like dithiol peptides that have the intrinsic ability to catalyze the reduction of protein disulfides. These thioredoxin-like dithiol peptides will generally have the characteristic of containing a pair of cysteine residues which form a redox-active disulfide. This example includes peptides, derived from natural sources or constructed synthetically, that include the same redox-active peptide sequence disclosed above, for example, as in E. coli thioredoxin, Cys-Gly-Pro-Cys-Lys, or analogous sequences from other thioredoxins such as that encoded for by T4 bacteriophage, Cys -Val-Tyr-Cys (Cys = cysteine, Val = valine, Tyr = tyrosine) (Soderberg, B-O, Sjoberg, B-M, Sonnerstam, U., and Branden, C-I [1978] Proc. Natl, Acad. Sci. USA, 75, 5827-5830). Other thioredoxin-like peptides include the class of seed proteins called purothionins that have intrinsic thioredoxin-like activity (Wada, K. and Buchanan, B.B. [1983] in "Thioredoxins, Structure and Function" [Gadal, P., ed], Editions du Centre National de la Recherche Scientifique).

. Examples of proteins containing disulfide crosslinks are insulin, proinsulin, bovine pancreatic ribonuclease (RNase), lysozyme, bovine pancreatic trypsin inhibitor, interferon, rennin, plasminogen activator, prolactin, human $\alpha$-1 trypsin inhibitor, Factor VIII, and the like.

The above proteins, and others containing disulfide crosslinks, can be used in their S-sulfonated form as starting material in the invention disclosed herein. When the S-sulfonated form of the protein is used, reduced thioredoxin is added to the folding process.

Materials and Methods used in the examples disclosed infra are as follows:

Materials: Bovine pancreatic ribonuclease A - (RNase), cytidine-2' :3'-cylcic monophosphate and oxidized and reduced dithiothreitol (DTT) were obtained from Sigma, St. Louis, MO. 3-(N-morpholine)-propane-sulfonic acid (MOPS) sodium salt, was the Ultrol grade from Calbiochem, San Diego, CA. Sephadex G-25-40 was also obtained from Sigma. All other chemicals used were reagent grade.

Thioredoxin is purified either from a commercial source of E. coli, strain B (Grain Processing Corp., Minneapolis, MN) or from any of a number of common strains of E. coli grown by standard procedures (Pigiet, V. and Conley, R.R. [1977] J. Biol. Chem., 252, 6367-6372). The protein is purified using standard procedures including chromatography on ion exchange and molecular seive columns (Williams, C. H., Zanetti, G., Arscott, L.D., and McAllister, J.K. [1967] J. Biol. Chem. 242, 5226-5231; McEvoy, M., Lantz, C., Lunn, C.A., and Pigiet, V. [1981] J. Biol. Chem. 256, 6646-6650).

Thioredoxin protein is assayed immunologically using quantitative rocket immunoelectrophoresis as described in McEvoy et al., supra.

Concentration and Activity of RNase A: Enzyme concentration of native RNase was determined using an extinction coefficient of $9800m^{-1}cm^{-1}$ measured at 277.5 nm (Hantgan, R.R., Hammes, G.G. and Scheraga, H.A. [1974] Biochemistry 13:3421-3431). The concentration of the fully reduced enzyme was determined at 275 nm using an extinction coefficient of $9200M^{-1}cm^{-1}$ - (Anfinsen, C.B., Haber, E., Sela, M. and White, F.H., Jr. [1961] Proc. Natl. Acad. Sci. USA 47:1309-1315).

The activity of RNase A was determined according to the procedure of Crook et al. (Crook, E.M., Mathias, A.P. and Rabin, B.R. [1960] Biochem. J. 74:234-238). The final assay mixture consisted of 0.1M MOPS, pH 7.0, 7mM cytidine-2' :3'-cyclic monophosphate and 10-30 $\mu$g/ml RNase A. The increase of the absorbance at 291 nm upon the hydrolysis of cytidine-2' :3'-cyclic monophosphate at 25°C was monitored.

Following are examples which illustrate the process of the invention, including the best mode. These examples should not be construed as limiting. All solvent mixture proportions are by volume unless otherwise noted.

Example 1--Denaturation and Refolding.

Reduction and denaturation of RNase was performed by incubating overnight 30 mg of the native enzyme in 1.5 ml of 0.1M Tris/HCl, pH 8.6 containing 0.15M DTT and 6M guanidine•HCl. The reduced RNase was separated from excess DTT and guanadine•HCl by column chromatography using a 1 cm x 24 cm Sephadex G-25 superfine column equilibrated and developed with 0.01N HCl. (Rudolph, R. and Fuchs, I. [1983] Hoppe-Seyler's Z. Physiol. Chem. 364, 813-820.) Fractions of 0.3 ml were collected and the absorbance at 280 nm was monitored. Those fractions containing RNase A were pooled, the concentration determined, and the material stored under argon frozen at -20°C.

Reoxidation of RNase A was initiated by diluting the reduced enzyme in 0.1M Tris, pH 7.4 with 1 mM EDTA containing various amounts of thioredoxin and/or oxidized DTT. At various times aliquots were assayed and the percentage of refolding calculated. The results were as follows:

Thioredoxin, oxidized DTT, and a mixture of both increased the rate of refolding of RNase as compared to air oxidation. The time required to obtain 50% reactivation (T $_{1/2}$) of RNase (310 $\mu$g/ml) was decreased approximately 15 fold as compared to air oxidation. For air oxidation the $T_{1/2}$ was 120 hr compared to 55 hr for 20 $\mu$M

thioredoxin, 45 hr for 50 $\mu$M, 35 hr for 100 $\mu$M and 8 hr for 500 $\mu$M. With thioredoxin, 100% of RNase activity was recovered after 30-90 hr while air oxidation would require at least 200 hr.

At lower concentrations of thioredoxin there was a lag in the reactivation of RNase between 10-20 hr. This lag was not observed in the presence of oxidized DTT. The most likely explanation is that DTT was more effective in some early kinetic step, perhaps an oxidation of disulfides inaccessible to the much larger thioredoxin molecule.

When oxidized DTT was used as the oxidant, similar results were observed. The $T_{1/2}$ for 20 mM DTT was 70 hr, while for 50 mM DTT it was 40 hr., and for 100 mM it was 30 hr. The values are comparable to those of thioredoxin at concentrations of 20, 50, and 100 $\mu$M. Thioredoxin is therefore 1000 times more effective than oxidized DTT. There is no observed increase in the rate of refolding using oxidized DTT at concentrations greater than 80mM. At these concentrations oxidized DTT is not very soluble and the reaction mixture may be saturated.

A mixture of thioredoxin and oxidized DTT was more effective than either component and no lag phase was observed. At a thioredoxin concentration of 20 $\mu$M the $T_{1/2}$ was lower by 15 hr with the addition of 20 $\mu$M DTT.

In summary, at low concentrations (i.e., 20$\mu$M), thioredoxin is more effective than DTT. A mixture of both is more effective than either component alone. At all concentrations of thioredoxin, the $T_{1/2}$ is decreased by an average of 10 hr with the addition of 20mM oxidized DTT. This suggests that DTT and thioredoxin may be influencing the oxidation of different disulfides. As the concentrations of either DTT or thioredoxin increase, the difference between the samples diminishes.

Example 2--Use of Thioredoxin and Oxygen to Refold RNase.

Renaturation of RNase was initiated by diluting the reduced enzyme in 0.1 M Tris, pH 7.5 with 1 mM EDTA to a final concentration of 340 $\mu$g/ml. Oxidized thioredoxin (100 $\mu$M: was added to half of the samples. All samples were stirred constantly at room temperature and a continuous stream of oxygen was run over the samples. The control samples were exposed to air. At various times aliquots were removed, assayed for RNase activity, and the percent of reactivation was calculated.

The time required for 50% reactivation of RNase ($T_{1/2}$) in the presence of 100 $\mu$M thioredoxin was decreased three fold with the addition of oxygen. The $T_{1/2}$ for 100 $\mu$M thioredoxin and air was 23 hr. With the addition of oxygen the $T_{1/2}$ was lowered to 8 hr. This is comparable to the $T_{1/2}$ of

500 $\mu$M thioredoxin in the presence of air. The effective concentration of thioredoxin can be reduced by a factor of five with the addition of oxygen. The oxygen effect does not occur in the absence of thioredoxin. There is an initial increase in the rate of refolding without thioredoxin, but the recovered activity levels off at 12% after 5 hr.

Example 3--Use of Thioredoxin and Thioredoxin Reductase to Refold RNase.

Thioredoxin reductase was obtained from IMCO (Stockholm, Sweden) or isolated according to the procedure of Pigiet and Conley (Pigiet, V. and Conley, R.R. [1977] J. Biol. Chem. 252, 6367-6372). NADP+ was obtained from Sigma (St. Louis, MO).

Refolding of RNase was initiated by diluting the denatured RNase in 0.1 M Tris, pH 7.5 or 0.05 M Tris, pH 9.0, each containing 1 mM EDTA. The regenerating system consisted of a catalytic amount of thioredoxin reductase and an excess (1 to 10 mM) of NADP+. The thioredoxin concentration remained constant at 100 $\mu$M. Aliquots were removed at various times and assayed for RNase activity.

In the presence of the above regenerating system of thioredoxin reductase and NADP+ there was an increase in the rate of refolding of RNase with 100 $\mu$M thioredoxin. The $T_{1/2}$ was decreased from 23 hr to 5-6 hr with thioredoxin reductase and either 0.1 or 1.0 mM NADP+.

Example 4--Thioredoxin Fragments $T_{1-37}$ and $T_{19-36}$.
(a) Production of $T_{1-37}$ by Cyanogen Bromide Cleavage

A sample of $\underline{E}$. $\underline{coli}$ thioredoxin was dialyzed in water for 12 hr at 4°C. Five ml was dried and resuspended in 70% formic acid. Cyanogen bromide (Sigma Chemical) was dissolved in 70% formic acid and added to thioredoxin in a 50-fold molar excess of methionine. The solution was purged with nitrogen and incubated at room temperature in the dark for 24 hr. At the completion of the cleavage reaction the solution was dried under nitrogen, resuspended in sodium acetate buffer and adjusted to pH 8.5 with ammonium hydroxide.

Samples were loaded onto a Waters $\mu$-Bondapak C-18 column (Trademark of Waters Associates, Inc., Milford, MA) attached to a Beckman Model 421 system (Trademark of Beckman Instruments, Inc., Fullerton, CA) monitored at 214 nm. The solvent system employed was 0.1% trifluoroacetic acid (Buffer A) and 0.08% trifluoroacetic acid in acetonitrile (Buffer B). A gradient from 0% to 60% B over 30 min was used to separate the peptides at a flow rate of 2 ml/min.

Thioredoxin was cleaved by CNBr into two fragments, $T_{1-37}$ and $T_{38-108}$, eluting at 44% and 51% buffer B, respectively. Amino acid analysis identified and confirmed the composition of both peptides. (Holmgren, A. and Reichard, P. [1967] Eur. J. Biochem. 2, 187-196). $T_{1-37}$ contained the active site of the enzyme. The two peptides recovered accounted for 69% of the starting material. Unreacted thioredoxin accounted for 12-15% of the loss, while HPLC separation may be responsible for the additional losses.

(b) Production of $T_{19-36}$ by Trypsin Cleavage

After HPLC separation, described above, $T_{1-37}$ was pooled, dried, and resuspended in sodium acetate buffer and adjusted to pH 8.0 with $NH_4OH$. An aliquot of trypsin (Sigma Chemical) was added to the incubation at 1% (w/w) of peptide concentration. The reaction mixture was incubated at 37°C for 1 hr. Separation of trypsin fragments was done by HPLC as for the cyanogen bromide fragments.

Trypsin digestion of the $T_{1-37}$ peptide yielded two peptides, $T_{4-18}$ and $T_{19-36}$, which were resolved by HPLC, eluting at 31% and 45% in buffer B, respectively. Amino acid analysis revealed that the species eluting at 31% B contained 15 amino acids and corresponds to the active site peptide, $T_{19-36}$. Incubation of 90 nmoles of $T_{1-37}$ produced 80 nmoles $T_{19-36}$ after separation by HPLC with a yield of 88%.

Example 5

Upon substituting the thioredoxin in Examples 1, 2 and 3 with the thioredoxin fragments $T_{1-37}$ or $T_{19-36}$ obtained in Example 4, there is obtained essentially the same results.

Example 6

Upon substituting oxidized thioredoxin or an oxidized thioredoxin-derived, or thioredoxin-like, dithiol peptide, alone, or in combination with oxygen or a thioredoxin reductase regenerating system, for the DTT or DTE in the examples of U.S. Patent 4,421,685, there is obtained an improved yield of the desired insulin.

Example 7

Upon substituting any disulfide protein or S-sulfonated disulfide protein for the bovine pancreatic ribonuclease of Examples 1, or 2, or 3, or 5, there are obtained comparable beneficial protein folding results.

Example 8

Upon reacting an unfolded protein whose disulfide crosslinks have been reduced, with a mixture comprising an effective protein folding amount of oxidized thioredoxin or an oxidized thioredoxin-derived, or thioredoxin-like, dithiol peptide and a thiol reducing agent selected from the group consisting of dithiothreitol and dithioerythritol, there is obtained an enhanced folding of said protein.

Example 9

Upon reacting an unfolded S-sulfonated disulfide protein with a mixture comprising an effective protein folding amount of reduced thioredoxin or a reduced thioredoxin-derived, or thioredoxin-like, dithiol peptide and a thiol reducing agent, for example, dithiothreitol or dithioerythritol, there is obtained an enhanced folding of said protein.

Example 10--Use of Thioredoxin to Refold Scrambled RNase

Scrambled RNase was produced by oxidation under denaturing conditions of the reduced and denatured RNase isolated as described above. The pooled fractions from the Sephadex G-25 column were made 6 M in guanidine•HCl and the pH adjusted to pH 8.6 with solid Tris. Oxygen was then bubbled through for several minutes and the sample was incubated at room temperature protected from light for 3-4 days. The amount of free thiol determined by the method of Ellman (Ellman, G.L. [1959] Arch. Biochem. Biophys. 82:70-77) was less than 0.05 mole per mole of RNase. The activity was about 5%, indicating that the majority of refolded RNase was in an incorrect and inactive form.

Reactivation of scrambled RNase was initiated by diluting the inactive RNase into 0.1 M Tris, pH 7.4 with 1 mM EDTA containing various amounts of oxidized thioredoxin preincubated with reduced DTT 30 min prior to addition. At various times, aliquots were assayed, and the percentage of reactivation was calculated by comparison of the specific activity with native RNase as previously described.

Thioredoxin efficiently catalyzes the rearrangement of disulfide bonds in scrambled, inactive RNase at pH 7.4 to yield a quantitative recovery of active enzyme. Pre-reduction of oxidized thioredoxin with reduced DTT was required to stimulate reactivation using this oxidized substrate. At 100 $\mu$M thioredoxin, the optimal level of DTT was 10 $\mu$M. Higher levels of DTT inhibited reactivation probably by shifting the protein thiol-disulfide balance toward the reduced form. The optimal oxidized thioredoxin to reduced DTT was 10:1. When this ratio remained constant and thioredoxin concentration was increased, an increase in the rate of reactivation was observed. The $T_{1/2}$ values for 150 $\mu$M and 200 $\mu$M thioredoxin were 2 hr and 1.5 hr respectively compared to 5 hr for 100 $\mu$M. The $T_{1/2}$ using 100 $\mu$M thioredoxin and 10 $\mu$M DTT was 5 hr, compared to 27 hr using thioredoxin in the refolding of reduced RNase. Reduced thioredoxin at a concentration of 10 $\mu$M was also effective in catalyzing the rearrangement of disulfide bonds in scrambled RNase with a $T_{1/2}$ of 8 hr. Neither air or DTT alone were effective in this reactivation reaction with a recovery of only 15% activity with air and 30% activity with DTT after 10 hr, compared to 75% reactivation with thioredoxin in the same time period.

The effective protein folding amount of reduced thioredoxin or a reduced thioredoxin-derived, or thioredoxin-like, dithiol peptide is about 5 to about 20 $\mu$M. Also, the effective protein folding amount of reduced and oxidized thioredoxin is, advantageously, maintained at a ratio of reduced to oxidized forms of about 1:1 to about 1:20.

Reduced thioredoxin is formed by preincubating oxidised thioredoxin and DTT in the folding mixture, as described above.

Example 11--Refolding of Sulfonated RNase with Thioredoxin

Thioredoxin efficiently catalyzed the refolding of sulfonated RNase at pH 8.0 to yield a quantitative recovery of active RNase. Sulfonated RNase was prepared by the method of Thannhauser and Scheraga (Thannhouser, T.W. and Scheraga, H.A. [1985] Biochemistry 24:7681-7688). Reduced thioredoxin was required and the optimal conditions were a 1:1 molar ratio of thioredoxin to the protein thiosulfonates (8:1 molar ratio thioredoxin to RNase). When the ratio was decreased to 1:2 thioredoxin to protein thiosulfonates, both the rate of reactivation and the final yield of RNase activity decreased. At a 1:1 ratio, the $T_{1/2}$ (time required for 50% recovery of activity) was 3.5 hr and the final yield of RNase was 100%. When thioredoxin was decreased two-fold, the $T_{1/2}$ increased to 8 hr and the recovery of RNase activity was only 65%. At a ratio of 1:20 thioredoxin to protein thiosulfonates, no reactivation above control was observed.

Example 12

Upon substituting the thioredoxin in Example 11 with the thioredoxin fragments $T_{1-37}$ or $T_{19-36}$ obtained in Example 4, or other thioredoxin-derived, or thioredoxin-like dithiol peptides in the reduced form, there is obtained essentially the same results.

## Claims

1. A process for producing a folded, disulfide-crosslinked protein, in vitro, which comprises reacting the corresponding unfolded, reduced protein with an oxidised dithiol peptide selected from oxidised thioredoxin and derivatives and analogues thereof.

2. A process for producing a folded, disulfide-cross-linked protein, in vitro, which comprises reacting the corresponding unfolded, reduced protein in the S-sulphonated form with a reduced dithiol peptide selected from reduced thioredoxin and derivatives and analogues thereof.

3. A process for producing a folded, disulfide-cross-linked protein, in vitro, which comprises reacting a scrambled protein containing incorrect disulfide cross-links with a reduced dithiol peptide selected from reduced thioredoxin and derivatives and analogues thereof.

4. A process according to claim 2 or claim 3, which comprises also the earlier step of preincubating oxidised thioredoxin and DTT, to give the reduced thioredoxin as the reduced dithiol peptide.

5. A process according to any of claims 2 to 4, wherein 5 $\mu$M to 20 $\mu$M of the reduced dithiol peptide are used.

6. A process according to any of claims 2 to 5, wherein the reduced dithiol peptide is used in combination with an oxidised dithiol peptide as defined in claim 1.

7. A process according to claim 6, wherein the ratio of reduced to oxidised dithiol peptide is 1:1 to 1:20.

8. A process according to any of claims 1, 6 and 7, wherein 5 $\mu$M to 500 $\mu$M of the oxidised dithiol peptide are used.

9. A process according to any of claims 1, 6, 7 and 8, wherein the oxidised dithiol peptide is used in combination with oxygen or a thioredoxin reductase regenerating system.

10. A process according to claim 9, wherein the thioredoxin reductase regenerating system comprises thioredoxin reductase and a nicotinamide adenine dinucleotide derivative.

11. A process according to claim 10, wherein the nicotinamide adenine dinucleotide derivative is the oxidised form of $NADP^+$.

12. A process according to any of claims 1 to 9, wherein the dithiol peptide is used in combination with a thiol oxidising agent.

13. A process according to claim 12, wherein the thiol oxidising agent is oxidised dithiothreitol or oxidised dithioerythritol.

14. A process according to any preceding claim, wherein the disulfide-cross-linked protein is insulin, proinsulin, bovine pancreatic ribonuclease -

(RNase), lysozyme, bovine pancreatic trypsin inhibitor, interferon, rennin, plasminogen activator, prolactin, human α-1 trypsin inhibitor or Factor VIII.

15. A process according to claim 14, wherein the disulfide-cross-linked protein is insulin or proinsulin.

16. A process according to any preceding claim, wherein the disulfide-cross-linked protein is in a crude cell extract or partially purified from a crude cell extract.

17. A process according to claim 16, wherein the cell is a recombinant microorganism.

18. A process according to any preceding claim, which comprises also the earlier step of cyanogen bromide cleavage of E. coli thioredoxin, to give the fragment containing residues 1 to 37 as the dithiol peptide.

19. A process according to claim 17, which comprises also the intermediate step of trypsin digestion of the residues 1 to 37, to give the fragment containing residues 19 to 36 as the dithiol peptide.

20. A process according to any of claims 1 to 17, wherein the dithiol peptide comprises the redox-active peptide sequence Cys-X-Y-Cys-Lys, wherein each of X and Y can be any of the 20 naturally-occurring amino-acids.

21. A process according to claim 20, wherein the redox-active peptide sequence is Cys-Gly-Pro-Cys-Lys.